# EUROPEAN PATENT APPLICATION

(11) **EP 1 493 413 A2**
(43) Date of publication of application: **05.01.2005**
(21) Application number: 04253889.2
(22) Date of filing: 29.06.2004
(51) Int. Cl.: A61F 13/15

(54) **Enhanced embossing and related methods**

(30) Priority: 30.06.2003 US 609745
(71) Applicant: McNeil-PPC, Inc., Skillman, New Jersey 08558 (US)
(72) Inventor: Disalvo, Anthony L., Bernardsville, New Jersey 07924 (US); Hull, Raymond J. Jnr., Hampton, New Jersey 08827 (US); Schmidt, Brian D., Little Falls, New Jersey 07424 (US)
(74) Representative: Mercer, Christopher Paul

(57) **Abstract**

An enhanced embossing pattern of an absorbent article having a substrate that has at least one embossed area and one unembossed area, wherein the embossed area has at least two sidewalls and a floor and a coating deposited in the embossed area, wherein the coating imparts visual contrast between the embossed area and the unembossed area of the substrate. Also included are related methods of making such absorbent articles.

## Description

### Field of the Invention

The invention relates to printing in an embossed area. In particular, the invention relates to printing in embossed areas of an absorbent article.

### Background of the Invention

Embossing methods for substrates are well known. Typically, embossing involves placing a substrate between two rollers. One roller may be hard (e.g., made from stainless steel) and the other may or may not be softer (e.g., made from rubber). Various designs and patterns, some of which are commercially available, are also known in the art. For example, US Pat. Nos. 6,136,413, 4,978,565, and US 2001/0008179 purport to disclose enhanced visual attractiveness to sheet material by various embossing methods. Further, U.S. Pat. No. 4,623,340 (Luceri) discloses products for absorbing body fluids that are provided with a pattern of depressed areas wherein the visual pattern contrast does not depend wholly upon the depth of the depressions or the degree of compression. This is accomplished by providing color contrast between the outer cover and the interior layer. Luceri describes that if the pattern of depressions is imposed upon the cover when the cover and the dark colored layer are in face-to-face relationship (i.e., coembossed), the depressed areas appear, as viewed from the external surface of the cover, as darker than the undepressed areas, i.e., visual contrast.

Some methods for embossing include printing the substrate. See for example, US Pat. Nos. 5,339,730 and 6,272,982.

Absorbent articles, such as, pantiliners, sanitary napkins, interlabial devices, adult incontinence devices, bandages, wipes, breast pads, shoe insoles, underarm pads and diapers are well known in the art. These articles typically have a fluid permeable body-facing side and fluid impermeable garment facing side. Additionally, such articles may include an absorbent layer for retaining fluids therebetween. Typically, the outer surface of the absorbent article is embossed or otherwise imposed with embossed patterns on the outer surface.

In particular, embossing may be done by any conventional method and results in at least one embossed area having a floor or valley and at least to two sidewalls extending from the floor to the top edge of the sidewalls that is co-extensive with the unembossed outer surface. To be aesthetically effective, such embossing must be relatively deep; e.g., the depressed areas must be permanently depressed to a degree, which represents a major portion of the thickness of the product. Shallow embossing is not visually effective. Unfortunately, the effect of extreme embossing may also be a source of discomfort for users of such an article so as to cause chafing, especially if contacting the thighs.

In addition, those skilled in the art have used specialty materials, e.g., thermoplastic materials in an attempt to provide visual contrast. Where thermoplastic materials are used, heat can be applied during embossing to the embossment. Such methods require additional energy and expense that are avoided by the materials of the present method.

Valley printing is a method of decorating or coating that includes not only the embossing of a texture, but also the transfer of an ink or other coating materials into the embossed area. Where ink is used, such decorating or coating is achieved by applying ink to the raised male surface of an embossing roll or plate in either single or multiple color schemes, and the transfer of that ink during the embossing or texturing process so that it is deposited into the embossed area of the article. Such a process as described above results in registration between the printing and the embossing. This results in a printed and three-dimensional effect to the article. This type of printing also forms contrast between the valley and the raised portion. In addition, properties, such as, wicking or hydrophobicity can also be added to the valley using this method.

It is desirable to provide visual contrast in personal products or consumer articles, such as those used in connection with the body. Heretofore, methods of valley printing have been used for surface coverings, such as, wallpaper, flooring, e.g., carpets, high pressure laminates, and films. Therefore, there is a need for valley printing of personal products or consumer articles. This invention fulfills that need.

### Summary of the Invention

The present invention provides an enhanced embossing pattern of an absorbent article comprising, consisting of, and/or consisting essentially of a substrate comprising at least one embossed area and one unembossed area, wherein the embossed area comprises, consists of, and/or consists essentially of at least two sidewalls and a floor; and a coating deposited in the embossed area, wherein the coating imparts visual contrast between the embossed area and the unembossed area of the substrate.

The present invention also provides an absorbent article having at least one enhanced embossing pattern, comprising, consisting of, and/or consisting essentially of a substrate comprising, consisting of, and/or consisting essentially of at least one embossed area and one unembossed area, wherein the embossed area comprises, consists of, and/or consists essentially of at least two sidewalls and a floor; and a coating deposited in the embossed area, wherein the coating imparts visual contrast between the embossed area and the unembossed area of the substrate.

The present invention additionally provides a method of making an enhanced embossment on an absorbent article comprising, consisting of, and/or consisting essentially of the steps of: providing a substrate, embossing the substrate with at least one embossment, depositing a coating in the at least one embossment to produce an enhanced embossment on the absorbent article.

The present invention also provides a method of making an absorbent article having at least one enhanced embossing pattern, comprising, consisting of, and/or consisting essentially of a substrate comprising at least one embossed area and one unembossed area, wherein the embossed area comprises at least two sidewalls and a floor; a coating deposited in the embossed area, wherein the coating imparts visual contrast between the embossed area and the unembossed area of the substrate.

The present invention additionally provides a method of making an absorbent article having an enhanced embossing pattern comprising, consisting of, and/or consisting essentially of providing a body facing surface having an outer surface comprising a plurality of embossments, wherein at least one of the embossments comprises, consists of, and/or consists essentially of a coating to provide at least one enhanced embossment.

### Brief Description of the Drawings

FIG. 1 is a plan view of a pantiliner according to the invention and
FIG. 2 is a cross sectional of the pantiliner taken along line 2-2 of FIG. 1.

### Detailed Description of the Invention

The present invention is directed to enhanced embossing patterns that are produced by coating an embossed area to provide visual contrast and methods of making such enhanced embossing patterns. Such patterns are useful in a variety of personal products or consumer articles, including, but not limited to, absorbent articles, hats, scarves, surgical masks, and surgical gowns. A non-limiting list of examples of absorbent articles is a sanitary napkin, a pantiliner, an incontinence device, an interlabial device, a tampon, a shoe insole, a breast pad, a diaper, and the like. In general, absorbent articles are used for absorbing body exudates.

In this invention, printing into the depressed areas of an embossed absorbent article with a contrasting material resulted in a dramatically enhanced surface. Depending upon the selection of the material printed onto the article, upon exposure to fluid, the contrasting material could direct the fluid flow, help retain the integrity and shape of the article, or appear to provide a separation of the fluid and the body contacting surface. The embossing and printing of the valley, sidewall, or both may take place in one step.

As used herein, the term "visual contrast" means any surface enhancement that produces a differentiation in the surface and is discemable by the naked eye, e.g., a three-dimensional effect. Non-limiting examples of visual contrast are deep embossing patterns and embossed patterns wherein the embossed area has a color difference compared to the un-embossed area.

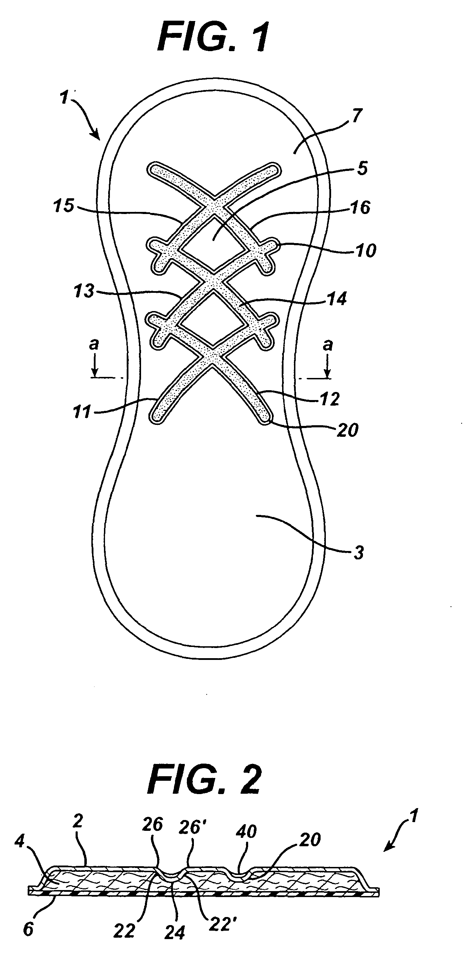

Figures 1-2 illustrate a single embodiment of the present invention. Absorbent article 1 is depicted as having an absorbent core 4, a cover 2 and a backsheet 6. Absorbent article has been embossed with a criss-cross pattern of embossments and a coating has been deposited on the floor of the embossed area 20 to provide visual contrasts with the unembossed area. Other embodiments as described later are also possible.

Looking at Figure 1, absorbent article 1 is shown in plan view. Cover 5 has body facing surface 7. Absorbent article 1 has been embossed such that embossed pattern 10 forms embossed area 20 in body facing surface 7. In this embodiment, embossed pattern 10 is formed by individual embossed areas 11-16, some of which intersect to form a criss-cross pattern. It is contemplated that any embossed pattern may be useful. Such embossed patterns may include non-intersecting embossed areas, any geometric shaped embossed areas, e.g., square, circle, triangle, waves, curves, leaves, petals, hearts, diamonds, and the like, logos, icons, animals, planets, cartoon, movie, television, radio, and print characters, public figures, and the like.

As seen in Figure 2, embossed area 20 has at least two sidewalls 22, 22' and a floor 24. Coating 40 has been printed or otherwise deposited in the embossed area 20 to impart visual contrast between the embossed area 20 and the unembossed area 5 of the substrate 3. In this embodiment, coating 40 covers floor 24. In another embodiment, sidewalls 22, 22' may be partially coated with coating 40 but upper edges 26, 26' of sidewalls 22, 22'are not coated. In still another embodiment, floor 24 and sidewalls 22, 22,' including upper edges 26, 26', are deposited with coating 40 (not shown). In still another embodiment (not shown), coating 40 fills the embossed area 20 such that the top of coating 40 is even with or protrudes from the unembossed area 5.

In the embodiment shown in Figures 1 and 2, floor 24 is represented as flat. In other embodiments, the floor may have a textured surface such as described in "Enhanced Embossing Application" filed concurrently herewith, having serial number USSN 10/ , which is commonly owned and incorporated herein by reference.

In this invention, the substrate is processed such that the embossing step and coating step may be done at a single station, i.e., during the same step.

In one embodiment, embossing and coating can be done using a single station that provides perfect registration of the coating in relation to the embossing, and provides the possibility of better penetration of the coating into the substrate. In this method, an external embossing sleeve having a pattern is outside the screen and internal back-up roll of a rotary screen coating device. Such a configuration provides embossing and coating on the tops of the bosses produced by the sleeve because of through holes in the sleeve that permit the coating material to be deposited on the substrate. In other words, coating only occurs on the areas of the substrate that have not been depressed by the embossing sleeve and are higher than the valleys. The solid backup roll can be made of metal.

While rolls are exemplified above, such a procedure may also be carried out using plates known to those skilled in the art.

In carrying out one method of the present invention, producing an enhanced embossment is accomplished by providing a substrate, embossing the substrate with at least one embossment, depositing a coating in the at least one embossment to produce an enhanced embossment. The coating may be deposited on the floor, on at least one sidewall, or both of the embossed area 20. The embossing step and the depositing step may be carried out using a single station device.

In carrying out another method of the present invention, making an article having at least one enhanced embossing pattern is accomplished by providing an article having a substrate comprising at least one embossed area and one unembossed area, wherein the embossed area comprises at least two sidewalls and a floor; and a coating deposited in the embossed area, wherein the coating imparts visual contrast between the embossed area and the unembossed area of the substrate. The embossing step and the depositing step may be carried out using a single station device.

The coating used to produce the visual contrast of the present invention may include, but is not limited to, a gel, an ink, a paint, tinted super absorbent polymer, an adhesive, starch, e.g., a binding agent, polyvinyl alcohol, and mixtures thereof. The ink may be a hydrophobic ink or a hydrophilic ink. Inks may include additives including, but not limited to, a polymer, a humectant, a surfactant, rheology modifier, and mixtures thereof. Other additives include glitter and texturing materials, e,.g., beads.

Additionally, the coating of the present invention may include additives useful for treating disease or infection, such as, medicaments or active compounds, e.g., antifungals and antibacterials, now known or developed in the future, and indicator additives useful for indicating various states, e.g., pH, infection, pregnancy, menopause, and the like.

In addition, a film or coating of the present invention can include an encapsulated essential oil, such as those described in "AN ABSORBENT ARTICLE HAVING ODER CONTROL PROPERTIES" filed concurrently herewith, having serial number USSN 10/ , which is commonly owned and incorporated herein by reference.

In one embodiment, the unembossed area is white and the coating 40 is white. Optionally, the coating may be a different shade than the unembossed substrate.

In one embodiment, the absorbent article has an absorbent layer, which has a body facing surface, and a backsheet, which has a garment facing surface. The absorbent layer may be made of any material used to absorb fluid or exudates. The material may have a three-dimensional structure or it may be substantially flat. In this embodiment, the body facing surface of the absorbent layer is embossed and a coating is deposited in the embossed area to provide visual contrast.

In another embodiment of the invention, the cover and absorbent core form a composite material, which is embossed and coated. In another embodiment, the composite includes a transfer layer. The composite may be joined by any conventional means including embossing, ultrasonic welding and by the application of adhesive between the layers. In these embodiments, the composite is embossed and a coating is deposited in the embossed area to provide visual contrast.

### Absorbent Material

The absorbent core or layer of the present invention may contain any known absorbent materials including, but not limited to, absorbent fibers such as cellulose fibers, including, but not limited to wood pulp, regenerated cellulose fibers, and cotton fibers, rayon fibers and the like; superabsorbent fibers or particles; other naturally occurring absorbent materials, such as sphagnum or peat moss; and other synthetic absorbent materials, such as foams and the like. The absorbent layer may also include one or more of the following: thermoplastic binder fibers, latex binder, perfumes, or odor-controlling compounds. The layer may optionally be any substrate, e.g., tissue, woven, nonwoven, netted material, lace, or foam.

For example, the absorbent material can be a fluffy batt cut from a relatively loose web of non-woven fibers having a relatively high absorptive capacity. While the absorbent material can have any shape or silhouette, it usually has an asymmetric configuration. The absorbent material may also be a fibrous batt having an integral densified layer. In such a case, if a backsheet is desired, the absorbent material is positioned on the backsheet of the absorbent article so that the densified layer adjoins the backsheet. The densified layer has relatively higher wettability and liquid retentivity than the rest of the aforesaid batt and usually is formed by slightly moistening one surface of the batt and thereafter compressing the moistened surface. The absorbent core or layer may also be formed from multiple layers, each having a different density such that the uppermost layer (closest to the body) is less dense than the outer (closest to the garment).

Additionally, the absorbent material can be an offline-formed, homogeneously mixed, air-laid layer, roll good laminate or any other offline-formed absorbent composite.

In one embodiment, the absorbent layer is made of absorbent material that is made from a layer of pulp. In another embodiment, superabsorbent polymer (SAP) is mixed with the pulp to form an absorbent composite. This composite may be condensed to form a dense, thin layer. One example of such a material is Novathin® available from Rayonier, Jesup, GA.

SAP are particles that are capable of absorbing many times, at least 10, more preferably 15, and still more preferably over 15, their weight in exudate, under a pressure of 0.5 psi. It should be noted that, in the context of the present invention, there is no restriction that the superabsorbent particles actually be particulate. This expression is intended to cover superabsorbent fibers, and other superabsorbent materials, whatever their form and shape. These superabsorbent particles generally fall into three classes, namely starch graft copolymers, cross-linked carboxymethylcellulose derivates and modified hydrophilic polyacrylates. Examples of such absorbent polymers are hydrolyzed starch-acrylonitrile copolymer graft copolymer, a neutralized starch-acrylic acid graft copolymer, a saponified acrylic acid ester-vinyl acetate copolymer, a hydrolyzed acrylonitrile copolymer or acrylamide copolymer, a modified cross-liked polyvinyl alcohol, a neutralized self-cross-linking polyacrylic acid, a cross-linked polyacrylate salt, carboxylated cellulose, and a neutralized cross-linked isobutylene-malasic anhydride copolymer. In one embodiment of the invention, the superabsorbent particle is a cross-linked polyacrylate salt.

The superabsorbent particles are incorporated into the absorbent core in an amount no greater than about 60% on a weight per weight basis. Preferably, they are incorporated in an amount between about 40% and about 0.5% on a weight per weight basis. All specific amounts are within the range are explicitly included in this disclosure. In the present context, 12% superabsorbent on "a weight per weight basis" is meant to mean 0.12 grams superabsorbent particles per 1 gram of all components comprising the absorbent core.

The absorbent layer of the present invention may be constructed according to conventional techniques, e.g., by air-laying a mixture of wood pulp fibers and superabsorbent material. All such conventional techniques are within the scope of the present invention. In one embodiment, an absorbent layer is as described in U.S. Pat. No. 5,866,242, which is herein incorporated by reference in its entirety.

The ratio of SAP to wood pulp may be varied over a wide range. If desired, a layer or multilayer of drylaid type material can be used as the absorbent material to form the absorbent core. The absorbent material may be made of a superabsorbent polymer (SAP) of the type used in the art and wood pulp fibers having the desired density.

The absorbent core may also include additional materials, such as, wetting agents, odor control material, wetness indicator material, materials for administering or delivering medicaments, such as encapsulated medicaments, and materials for maintaining skin moisture, such as encapsulated moisturizers.

### Cover

Although not required, the absorbent article of the present invention may include a cover overlaying the absorbent material. The exterior of the cover would then form the body-facing surface of the absorbent article. The cover may be formed from any fluid pervious material that is comfortable against the skin and permits fluid to penetrate to the absorbent core, which retains the fluid. The cover should retain little or no fluid to provide a relatively dry surface next to the skin when in use. A variety of cover materials are known in the art, and any of these may be used. For instance, the cover may be a fibrous non-woven fabric made of fibers or filaments of polymers, such as polyethylene, polypropylene, polyester, or cellulose, and combinations thereof. Alternatively, the cover may be formed from an apertured polymeric film. The thickness of the cover may vary from about 0.001 to about 0.062 inch, depending on the material chosen.

Generally, the optional cover is a single sheet of material having a width sufficient to form the body-facing surface of the absorbent article. Preferably, the cover is longer and wider than the absorbent core.

### Transfer layer

The absorbent article of the present invention may include a transfer layer. The transfer layer may be made of any known material that will take up fluid and then distribute and release it to an adjacent absorbent core or layer for storage. Preferred transfer layers have a relatively open structure that allows for movement of fluid within the layer. Suitable materials for such transfer layers include any water dispersible material including fibrous webs, water dispersible resilient foams, and the like.

The transfer layer is able to accept fluid and allow passage of the fluid through its mass to be absorbed by an adjacent absorbent core. Thus, transfer layers that are made of hydrophobic, nonabsorbent fibers may be able to accept large volumes of fluid into interfiber void spaces while the fibers themselves do not absorb any significant quantities of fluid. Likewise, open-celled foam structures that are made from nonabsorbent materials may also absorb fluid into the cells of the foam. The walls of the cells, however, do not absorb any fluid. The cumulative spaces within the transfer layer, i.e., the interfiber void spaces in the fibrous transfer layer or the open cells in the foam transfer layer, function much like a container to hold fluid.

Transfer layers that are made from webs of mostly absorbent fibers absorb the fluid as it enters the structure and do not distribute it throughout the rest of the structure as efficiently as webs containing non-absorbent materials. Preferred transfer layer fibrous webs include nonabsorbent materials to provide void volume and to allow for free movement of fluid through the structure.

### Backsheet

The backsheet of the present invention is a body fluid impervious material, typically referred to as a "barrier," at least substantially impermeable to liquids, and its exterior forms the garment-facing surface of the absorbent article. The backsheet may be any thin, flexible, body fluid impermeable material, such as a polymeric film, e.g., polyethylene, polypropylene, or cellophane, or a normally fluid pervious material that has been treated to be impervious, such as impregnated fluid repellent paper or non-woven material, including non-woven fabric material, or a flexible foam, such as polyurethane or cross-linked polyethylene. The thickness of the backsheet when formed from a polymeric film typically is about 0.001 to about 0.002 inch.

Optionally, the backsheet may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet.

### Adhesives

Any construction adhesive may be used to attach the layers into a single absorbent article. For example, in one embodiment, the body facing layer is attached to the outer shell with adhesive HL 1491 available from H.B Fuller and Company (St. Paul, MN). The adhesive may be applied by any method known to those skilled in the art.

Additionally, the absorbent core may be coated with a fluid permeable or impermeable material in a discrete pattern. Examples of this type of coating can be found in co-pending application "ABSORBENT ARTICLE INCLUDING IN SITU COVER" filed concurrently herewith, having serial number USSN 10/ , which is commonly owned and incorporated herein by reference

Secure attachment of absorbent article of the claimed invention to the garment contributes to maintaining the feeling of the user that the absorbent article and the garment are one in the same, i.e., permits the absorbent article to move with the underwear.

The absorbent article of the present invention may be applied to the crotch by placing the garment-facing surface against the inside surface of the crotch of the garment. Various methods of attaching absorbent articles may be used. For example, chemical means, e.g., positioning adhesive, and mechanical attachment means, e.g., clips, laces, ties, and interlocking devices, e.g., snaps, buttons, VELCRO (Velcro USA, Inc., Manchester, NH), zipper, and the like are examples of the various options available to the artisan.

Positioning adhesive may include pressure sensitive adhesive that is applied as strips, swirls, or waves, and the like. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include adhesives based on the following: emulsion or solvent-borne adhesives of natural or synthetic polyisoprene, styrene-butadiene, or polyacrylate, vinyl acetate copolymer or combinations thereof; hot melt adhesives based on suitable block copoylmers - suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include Kraton® elastomers from Shell Chemical Company, Vector® elastomers from Dexco, Solprene® from Enichem Elastomers and Stereon® from Firestone Tire & Rubber Co.; hot-melt adhesive based on olefin polymers and copolymers where in the olefin polymer is a terpolymer of ethylene and a co-monomers, such as vinyl acetate, acrylic acid, methacrylic acid, ethyl acrylate, methyl acrylate, n-butyl acrylate vinyl silane or maleic anhydride. Commercial examples of these types of polymers include Ateva® ( polymers from AT plastics), Nucrel® ( polymers from DuPont), Escor (from Exxon Chemical).

Where adhesive is used, a release strip may be applied to protect the adhesive on the absorbent article prior to attaching the absorbent article to the crotch. The release strip can be formed from any suitable sheet-like material adheres with sufficient tenacity to the adhesive to remain in place prior to use but which can be readily removed when the absorbent article is to be used. Optionally , a coating may be applied to release strip to improve the ease of removabilty of the release strip from the adhesive. Any coating capable of achieving this result may be used, e.g., silicone.

The adhesive applied to the garment facing side of the absorbent article may be any adhesive known in the art. As a non-limiting example, pressure sensitive adhesive strips, swirls, or waves may be applied to help maintain the absorbent article in place. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include rapid setting thermoplastic "hot melt," rubber adhesives, two-sided adhesive tape, and the like.

The present invention may be used in making any shaped absorbent article. For example, pantiliners for use with thong-type underwear may be made using this invention. Additionally, the absorbent article may be tinted or colored, e.g., black, red, blue, and any color or mixture thereof, to offer discretion with the users undergarments.

The absorbent article may be packaged as unwrapped absorbent articles within a carton, box or bag. The consumer withdraws the ready-to-use article as needed. The absorbent article may also be individually packaged (each absorbent article encased within an overwrap).

Also contemplated herein include asymmetrical and symmetrical articles having parallel longitudinal edges, dog bone- or peanut-shaped, circuvlar, oval and the like.

An absorbent article of the present invention may be used with conventional underwear or may be shaped to conform to thong garments. As used herein, the term thong includes, but is not limited to, thong underwear, thong swimming suit bottom, G-strings, Rio cut underwear, Rio cut swimming suit bottom, Brazilian cut underwear, Brazilian cut swimming suit bottom, and any other garment that exposes the buttocks, having a narrow strip of fabric or a cord that passes between the thighs supported by a waistband, a waist cord, belt or the garment itself.

Additionally, articles, particularly absorbent articles, of the present invention may be flushable, that is discardable in a toilet, urinal, or other flushing device made for the purpose of receiving urine or other body exudates and transporting it through a public or private sewage or plumbing system. Flushable articles may be made of biodegradable materials, i.e., those materials that are capable of being broken down especially into innocuous products by the action of living things (such as microorganisms). In addition, flushable articles may break apart, i.e., the article or element exhibits visible changes after being flushed down a standard toilet; the changes may include any visible failures to the integrity of the article or element, such as, holes, slits, shreds; breaking apart into smaller sections; dissolving; or a combination thereof.

From the foregoing description, one skilled in the art can ascertain the essential characteristics of this invention, and without departing from the spirit and scope thereof, can make various changes and modifications. Embodiments set forth by way of illustration are not intended as limitations on the variations possible in practicing the present invention.

## Claims

1. An absorbent article having at least one enhanced embossing pattern, comprising:
a substrate comprising at least one embossed area and one unembossed area, wherein the embossed area comprises at least two sidewalls and a floor;
a coating deposited in the embossed area, wherein the coating imparts visual contrast between the embossed area and the unembossed area of the substrate.

2. An absorbent article of claim 1, wherein the coating is deposited on the floor of the embossed area.

3. An absorbent article of claim 1, wherein the coating is deposited on at least one sidewall of the embossed area.

4. An absorbent article of claim 1, wherein the coating is deposited on the floor area, at least one of the sidewalls of the embossed area, or both.

5. An article of any one of claims 1 to 4, wherein the coating is an ink, a paint, a tinted superabsorbent polymer, an adhesive, a binding agent, polyvinyl alcohol or a mixture thereof.

6. An absorbent article of claim 5, wherein the ink comprises an additive which is a polymer, a humectant, a surfactant, a rheology modifier or a mixture thereof.

7. An absorbent article of claim 5 or claim 6, wherein the ink is a hydrophobic ink.

8. An absorbent article of any one of claims 1 to 7, wherein the coating is a different shade than the unembossed substrate.

9. An absorbent article of any one of claims 1 to 8, wherein the absorbent article is a surgical mask, a surgical gown or a wipe.

10. An absorbent article of any one of claims 1 to 8, wherein the absorbent article is a sanitary napkin, a pantiliner, an incontinence device, an interlabial device, a tampon, a shoe insole, a breast pad, a diaper, a surgical mask, a surgical gown or a wipe.
